Europäisches Patentamt

**European Patent Office**

Office européen des brevets

(19)

(11) Numéro de publication: **0 055 751**
**B1**

# FASCICULE DE BREVET EUROPEEN

(12)

(45) Date de publication du fascicule du brevet:
**31.10.84**

(21) Numéro de dépôt: **81902035.5**

(22) Date de dépôt: **08.07.81**

(86) Numéro de dépôt international:
**PCT/FR 81/00090**

(87) Numéro de publication internationale:
**WO 82/00203 (21.01.82** Gazette 82/3)

(51) Int. Cl.³: **G 01 N 33/54, G 01 N 33/58**

(54) **PROCEDE DE DETECTION ET DE DOSAGE D'UNE SUBSTANCE BIOLOGIQUE PAR ERYTHROADSORPTION.**

(30) Priorité: **09.07.80 FR 8015293**

(43) Date de publication de la demande:
**14.07.82 Bulletin 82/28**

(45) Mention de la délivrance du brevet:
**31.10.84 Bulletin 84/44**

(84) Etats contractants désignés:
**CH DE GB LI**

(73) Titulaire: **INSTITUT PASTEUR, 25-28, rue du Docteur Roux, F-75724 Paris Cedex 15 (FR)**

(72) Inventeur: **AVRAMEAS, Stratis, 1, rue Sarasate, F-75015 Paris (FR)**
Inventeur: **GUESDON, Jean-Luc, 12, rue du Hameau, F-75015 Paris (FR)**

(74) Mandataire: **Phélip, Bruno et al, c/o Cabinet Harlé & Phélip 21, rue de la Rochefoucauld, F-75009 Paris (FR)**

(56) Documents cités:
**EP - A - 0 041 426
DE - A - 2 732 554
FR - A - 1 516 640
US - A - 4 130 634
US - A - 4 228 237**

Chemical ABstracts, volume 81, no. 11, publié le 16 septembre 1974, (Columbus Ohio, US) S. Lemieux et al. "Local hemolysis plaque assay using a new method of coupling antigens on sheep erythrocytes by glutaraldehyde", voir page 335, abrégé no. 61897s, Immunochemistry 1974, 11(5), 261-9 (Eng.) cité dans le demande
The Journal of Histochemistry and Cytochemistry, volume 27, no. 8, 1979, Jean-Luc Guesdon et al. "The use of avidin-biotin interaction in immunoenzymatic

(56) Documents cités: (suite)
techniques", pages 1131-1139 voir le document en entier cité dans la demande
Chemical Abstracts, volume 91, no. 25, publié le 17 décembre 1979 (Columbus Ohio, US) voir page 491, abrégé 209046t S.M. Costello et al. "Enchancement of immune cellular agglutination by use of an avidin-biotin system" Clin. Chem. (Winston-Salem, N.C.) 1979, 25(9), 1572-80, (Eng.)

## Description

La présente invention concerne un procédé de mise en évidence et de dosage d'une substance biologique par érythroadsorption.

Les techniques biologiques de localisation, de mise en évidence et de dosage de substances biologiques sont nombreuses. On peut citer par exemple les techniques immunoenzymatiques (dosages immunoenzymatiques ou détection histochimique), l'immunofluorescence (dosage immuno-fluorimétrique ou détection histochimique), la technique radioimmunologique, la technique d'hémagglutination et similaires.

Ces techniques mettent en oeuvre, à titre de réactifs, des substances marquées par une enzyme, un fluorochrome ou un radioisotope ou des globules rouges sensibilisés.

La technique d'hémagglutination pour la détermination d'anticorps consiste à mélanger des globules rouges sensibilisés par un antigène avec une solution contenant l'anticorps à déterminer ou à doser. Cette technique est effectuée en phase liquide. Pour plus de détails au sujet de la technique d'hémagglutination on pourra se reporter à l'ouvrage ci-après cité à titre de référence: »Handbook of experimental immunology« 3 vol. Ed. D. M. WEIR (1978) Blackwell Scientific.

On pourra également citer, à titre de documents illustrant l'utilisation d'érythrocytes sensibilisés dans des procédés de détection ou de dosage d'antigènes ou d'anticorps, les documents ci-après:

— la demande de brevet DE-B-2 732 554 qui concerne un système indicateur pour les réactions anticorps-antigènes, présenté sous la forme d'une matrice de gel. Ce système est constitué d'érythrocytes sur lesquels sont couplés des anticorps ou des antigènes en présence d'un tampon spécifique et d'un complément. Ce système fait donc intervenir des érythrocytes sensibilisés par un anticorps ou un antigène.

— le brevet US-A-4 130 634 qui concerne un procédé pour détecter la présence d'un antigène particulier dans une composition. Ce procédé consiste à mettre en contact des compositions avec des érythrocytes sur lesquels sont couplés des anticorps spécifiques dudit antigène, à ajouter un agent de développement comprenant un anticorps réactif avec ledit antigène, à ajouter du complément et à noter la lyse desdits érythrocytes comme une indication positive de la présence dudit antigène.

— le résumé des Chemical Abstracts vol. 81 n° 11 du 16 Septembre 1974, qui concerne le dosage en plaque par hémolyse locale avec des érythrocytes de mouton sensibilisés par des antigènes.

On notera également que l'on a proposé de soumettre les érythrocytes à un traitement préalable à l'aide d'un ou plusieurs agents tannants avant la sensibilisation. A cet effet on pourra se référer au brevet FR-A-1 515 640.

On a maintenant trouvé un nouveau procédé de dosage de substance biologique qui met en oeuvre, à titre de réactif de dosage, le produit de couplage d'un ligand spécifique de la substance à doser et d'un anticorps anti-érythrocytes, et des érythrocytes à titre de révélateur. Le procédé de l'invention permet d'éviter l'utilisation d'érythrocytes sensibilisés par des anticorps ou des antigènes.

Le procédé selon l'invention est un procédé de détection et de dosage d'une substance biologique par érythroadsorption qui consiste:

1) à immobiliser une substance ayant une affinité de fixation pour la substance biologique à doser;
2) à faire incuber cette substance avec le milieu liquide contenant la substance biologique à doser;
3) à faire incuber, après lavage, le milieu réactionnel résultant avec le produit de couplage d'un ligand spécifique de la substance à doser et d'un anticorps anti-érythrocytes;

4) à ajouter des érythrocytes; et
5) à déterminer l'érythroadsorption.

Le procédé selon l'invention est approprié pour doser, à titre de substance biologique, des antigènes, des anticorps, des haptènes, des hormones, des immuno-globulines et autres substances d'intérêt biologique.

La substance ayant une affinité de fixation vis-à-vis de la substance biologique à doser peut être toute substance capable de se fixer de façon spécifique avec ladite substance biologique. Par exemple, si la substance biologique à doser est un anticorps, la substance ayant une affinité de fixation sera alors un antigène et réciproquement.

La substance ayant une affinité de fixation pour la substance biologique à doser est immobilisée sur un support quelconque par des techniques classiques.

A titre de support, on peut utiliser n'importe quel support insoluble capable d'être mis sous forme de récipient. A titre de substance constitutive de tels supports, on peut utiliser la cellulose et ses dérivés, le polyacrylamide, les polyméthacrylates d'alkyle et autres polymères d'origine naturelle ou synthétique ainsi que le verre.

On utilise avantageusement des microplaques pour immobiliser la substance ayant une affinité pour

2

la substance biologique à doser, par exemple des microplaques en polystyrène. On a constaté que des microplaques présentant des cavités à section en U ou en V étaient les mieux appropriées.

Le produit de couplage mis en oeuvre à titre de réactif de dosage selon l'invention est le produit de couplage d'un ligand spécifique de la substance à doser et d'un anticorps anti-érythrocytes.

Par »ligand spécifique« on désigne dans la présente description toute substance soluble qui peut réagir de façon spécifique avec la substance ayant une affinité pour la substance biologique à doser ou avec la substance biologique elle-même.

Par »substance soluble« on désigne dans la présente description toute substance soluble dans les milieux couramment utilisés pour les réactions biologiques. Il peut s'agir de milieux aqueux, tels que les milieux physiologiques, ou des mélanges des milieux aqueux et organiques.

De plus, le ligand spécifique mis en oeuvre doit être tel que le produit de couplage ligand spécifique-anticorps anti-érythrocytes soit soluble en milieu aqueux.

Par »milieux aqueux« on désigne selon l'invention les milieux aqueux, tamponnés ou non, couramment utilisés dans le domaine biologique, tels que les solutions tampon phosphate, les solutions tampons contenant un détergent, tel que le »Tween« ou de la gélatine, la sérumalbumine bovine, la lactalbumine bovine et autres substances habituellement mises en oeuvre dans de tels domaines.

Les ligands spécifiques répondant à une telle définition sont notamment les anticorps, les antigènes macromoléculaires, les haptènes, les hormones et leurs récepteurs et substances similaires. Parmi les ligands spécifiques cités ci-dessus, on utilise le plus communément les anticorps et les antigènes.

Le ligand capable de réagir avec des érythrocytes est une substance qui possède des sites de reconnaissance de déterminants spécifiques des érythrocytes ou des substances fixée sur des érythrocytes. Dans la demande de brevet FR-A-8 011 470 on a décrit un produit de couplage entre une lectine et un ligand spécifique et ses applications dans le domaine biologique, notamment pour le dosage de substances biologiques dans lequel le marqueur peut être des globules rouges.

On/eut utiliser à titre de ligand capable de réagir avec des érythrocytes, normaux ou modifiés, une substance autre qu'une lectine, répondant à la définition ci-dessus. A titre d'exemples de tels ligands, on peut citer l'avidine, la biotine et produits analogues.

On indiquera que l'avidine et la biotine sont deux substances qui interagissent fortement par voie non covalente. On a déjà mis à profit cette forte interaction dans les techniques immunoenzymatiques telles que la technique dite pont avidine-biotine ou la technique mettant en oeuvre une enzyme marquée à l'avidine. A cet effet, on pourra se référer à l'article de GUESDON et al. (The Journal of Histochemistry and Cytochemistry vol. 27, n° 8, p. 1131—1139, 1979).

Quand on utilise de l'avidine comme ligand capable de réagir avec les érythrocytes, on/ent utiliser des érythrocytes modifiés, c'est-à-dire des érythrocytes porteurs de biotine et réciproquement.

De la même façon, on pourra utiliser à titre de ligand capable de réagir avec des érythrocytes, toute substance qui pourra interagir avec une substance fixée sur les érythrocytes. Ainsi, aux fins de l'invention, on désigne par »érythrocytes« aussi bien des érythrocytes normaux que des érythrocytes modifiés, c'est-à-dire protant une substance spécifique du ligand mis en oeuvre et qui est défini de façon générale comme un ligand capable de réagir avec des érythrocytes.

La modification des érythrocytes se fait selon des moyens classiques à la portée de l'homme de l'art, c'est-à-dire par des techniques de couplage couramment utilisées dans le domaine biologique (voir l'article de GUESDON et al précédement cité).

On indiquera que la biotine peut être fixée sur les érythrocytes à l'aide du biotinyl-N-hydroxysuccinimide selon le mode opératoire décrit dans l'article de JASIEWICZ et al [Exp. Cell. Res. 1976, 100, 213—217].

Le couplage ligand spécifique-ligand capable de réagir avec des érythrocytes est réalisé par l'intermédiaire d'un agent de couplage approprié, tel que par exemple le glutaraldéhyde et la benzoquinone. Le couplage est avantageusement réalisé par liaisons covalentes. Il est réalisé selon un procédé analogue à ceux utilisés pour le couplage des protéines.

Par exemple, on peut réaliser le couplage par un procédé similaire à celui décrit pour l'obtention de conjugués anticorps-enzyme dans Scand. J. Immunol., vol. 8, supp 7, p. 7—23, 1978. Un tel procédé de couplage consiste à mettre en contact, en une ou plusieurs étapes, un agent de couplage avec les substances à coupler.

On peut également utiliser comme agent de couplage la benzoquinone. A titre de document illustrant la technique de couplage à la benzoquinone, on peut citer le brevet FR-A-7 537 392 (publié sous le N° 2 337 107).

La détermination de l'érythroadsorption peut se faire de plusieurs façons. Par exemple, on peut constater visuellement que les globules rouges sont adsorbés à la surface du support sur lequel a été immobilisée la substance ayant une affinité de fixation pour la substance biologique à doser. Dans ce cas, le procédé de l'invention permet d'identifier dans un liquide biologique donné une substance biologique particulière. Si, au contraire, le liquide biologique ne contient pas la substance biologique particulière, les érythrocytes ne sont pas adsorbés et forment un culot au fond du récipient, par exemple des puits de microplaques. Le procédé de l'invention permet aussi de déterminer quantitativement une substance biologique donnée. A cet effet, on élimine les globules rouges n'ayant pas réagi, par exemple par aspiration à la pipette. Puis on lyse les érythrocytes adsorbés, par exemple à l'eau distillée, et on dose les substances libérées par les globules rouges, par exemple l'hémoglobine ou les substances artificiellement introduites par l'expérimentateur, par spectrophotométrie à 403 nm, ce qui permet d'automatiser entièrement ce procédé de dosage.

L'hémoglobine peut également être dosée par une réaction enzymatique. Par exemple, on peut utiliser un des substrats de la peroxydase, tels que l'ortho-dianisidine ou l'ortho-phénylène-diamine. La lecture se fait aussi par spectrophotométrie à 400 nm pour l'ortho-dianisidine et à 492 nm pour l'ortho-phénylène-diamine.

La quantité de substances libérées par les globules rouges, par exemple la quantité d'hémoglobine libérée, est proportionnelle à la quantité de la substance à doser, ce qui permet d'obtenir, par exemple, le dosage d'un antigène ou d'un anticorps présent dans l'échantillon en se référant à une gamme étalon d'hémolyse des globules rouges établie dans les mêmes conditions.

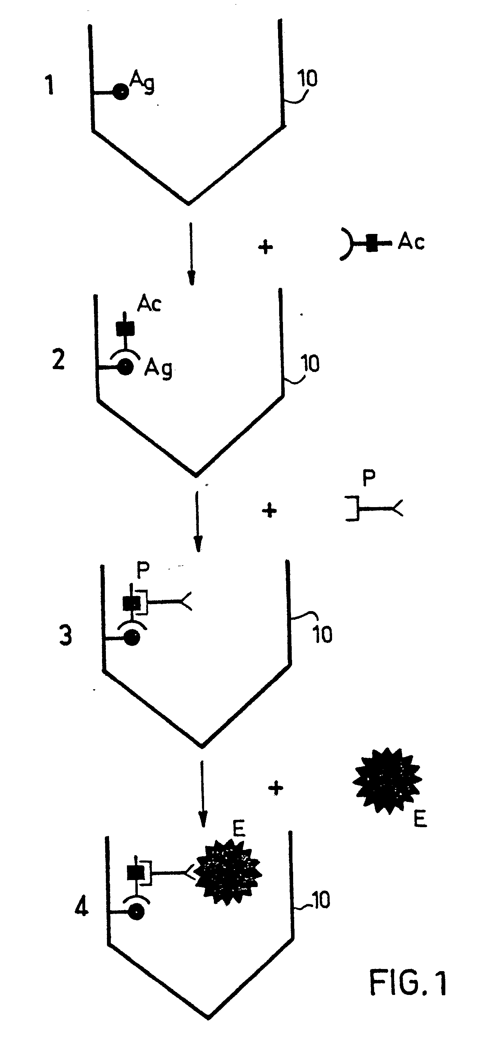

Le procédé selon l'invention est particulièrement approprié pour le dosage des anticorps et des antigènes et sera décrit plus en détail ci-après, sans en limiter pour autant la portée, en référence à la figure unique annexée, sur laquelle est représenté le schéma réactionnel du procédé de l'invention.

Sur la figure unique, (1) représente la première étape du procédé de l'invention au cours de laquelle on immobilise sur le support (10) des antigènes (Ag). Dans le cas particulier de la figure, le support (10) représente un puits en V d'une microplaque. L'immobilisation des antigènes, qui constituent dans ce cas particulier la substance ayant une affinité pour la substance biologique à doser, à savoir l'anticorps, est réalisée par exemple par adsorption passive ou, si nécessaire, par liaison covalente selon la nature du support.

L'étape (2) consiste en une incubation de l'antigène immobilisé avec le liquide biologique contenant l'anticorps (Ac) à doser, par exemple le sérum à titrer. Après cette étape d'incubation, au cours de laquelle l'antigène (Ag) interagit avec l'anticorps (Ac) correspondant, s'il est présent dans la sérum à tester, on lave le support à l'aide d'une solution tampon, par exemple une solution de tampon phosphate contenant éventuellement un détergent tel que le »Tween« dénommé ci-après PBS ou PBS-Tween.

L'étape (3) du procédé de l'invention consiste à incuber le support résultant de l'étape (2) avec un produit de couplage ligand spécifique anticorps anti-érythrocytes (P). Dans ce cas particulier, le ligand spécifique est un anticorps dirigé contre les immunoglobulines de l'espèce humaine ou animale du sérum à titrer. Après cette incubation, on lave le système résultant comme décrit ci-dessus pour éliminer le produit de couplage n'ayant pas réagi. Puis on ajoute des érythrocytes (E), qui sont adsorbés par le produit de couplage seulement si le sérum à titrer contient l'anticorps correspondant à l'antigène immobilisé, sinon les érythrocytes ne sont pas fixés et tombent au fond du récipient.

Dans son application en clinique humaine, le procédé de l'invention permet donc de détecter rapidement la présence dún anticorps donné le sérum dún malade.

Le dosage quantitatif, c'est-à-dire la détermination de la quantité d'anticorps contenue dans le sérum, sera aisément effectué comme décrit ci-dessus, c'est-à-dire par lyse des érythrocytes et détermination de la quantité de substance libérée par ces érythrocytes, par exemple la quantité d'hémoglobine.

On utilise avantageusement selon l'invention des globules rouges de mouton pour l'érythroadsorption. Etant donné que les sérums présentent d'une espèce à l'autre des réactions croisées et qu'ils contiennent souvent des anticorps anti-globules rouges, par exemple des anticorps anti-globules rouges de mouton, il faudra prendre soin d'absorber avec des globules rouges le sérum à tester pour éviter les réactions faussement positives. Bien qu'une telle technique fasse appel à des moyens connus de l'homme de l'art, on indiquera ci-après certaines de ses variantes de mise en oeuvre.

Dans le cas où l'anticorps ou l'antigène à doser n'est pas thermolabile, cette absorption pourra être effectuée en soumettant le sérum aux étapes ci-après qui consistent:

a) à décomplémenter le sérum, c'est-à-dire le chauffer à 56°C environ pendant environ 30 minutes;
b) à ajouter un excès de globules rouges de mouton audit sérum, par exemple pour obtenir une concentration de 10% de globules rouges;
c) à laisser le sérum à la température ambiante pendant environ 1 heure sous agitation douce;
d) à centrifuger pour récupérer le sérum absorbé et éliminer le culot de globules rouges.

Dans le cas où l'antigène ou l'anticorps à doser est thermolabile, le sérum ne sera pas décomplémenté mais il sera absorbé par des globules rouges, par exemple de mouton, traités par un agent tannant, de préférence le glutaraldéhyde.

Le procédé selon l'invention est un procédé simple et sensible, particulièrement approprié pour la détection ou le dosage des anticorps et des antigènes. Sa sensibilité est telle qu'elle permet de déceler un antigène présent à une concentration de l'ordre du nanogramme par millilitre.

Il est entièrement réalisé en phase solide; sa mise en oeuvre ne requiert aucune étape de séparation, centrifugation, filtration, autre que celle imposée par le support d'immobilisation de l'un des réactifs (par lavage du support).

Le procédé de l'invention va maintenant être illustré par les exemples non limitatifs ci-après.

On a indiqué ci-après la provenance des produits utilisés dans les exemples:

— des anticorps spécifiques isolés par passage de sérums entiers sur des immunoadsorbants correspondants préparés par immobilisation d'un antigène spécifique sur des billes de polyacrylamide agarose activées avec du glutaraldéhyde selon la technique décrite dans J. Immunol. Meth., 1976, 11, 129—133. Les immunsérums de mouton anti IgE humaines et les immunsérums de lapin anti IgC ou les anti-érythrocytes de mouton ont été obtenus par hyperimmunisation, d'animaux avec l'antigène correspondant dans l'adjuvant complet de Freund (Difco Laboratories Detroit USA) en utilisant la technique décrite dans Immunochemistry 1969, 6, 43—53.

Les anticorps anti-globules rouges de mouton ont été isolés à partir des immunsérums de lapin avec un copolymère à base glutaraldéhyde de sérumalbumine bovine et des stromas cellulaires selon le procédé décrit dans Immunochemistry, 1969, 6, 53—66. Les anticorps marqués par une enzyme utilisés dans l'exemple comparatif ont été préparés selon la technique décrite dans Immunochemistry 1969, 6, 43—53.

— de la sérumalbumine bovine vendue par POVITE (Amsterdam, Hollande);
— une solution aqueuse de glutaraldéhyde à 25% fournie par Taab Laboratoires (Reading, UK);
— du »Tween« et de la biotine fournis par Merck—Schuchardt (Darmstadt, Allemagne Fédérale);
— l'avidine a été préparée à partir de blanc d'oeuf et par chromatographie échangeuse d'ions sur colonne DEAE-cellulose;
— le DNA du thymus de veau a été fourni par Sigma Chemical Co (St-Louis, USA);
— la concanavaline A et le méthyl-alpha-D-mannoside ont été fournis par Industrie Biologique Française (Pharmindustrie).

## Exemple 1

### Dosage d'anticorps anti-DNA

Dans cet exemple, on a utilisé comme produit de couplage ligand spécifique-ligand capable de réagir avec des érythrocytes le produit de couplage d'anticorps anti-immunoglobulines humaines et d'anticorps anti-globules rouges de mouton obtenu par le procédé de couplage en une seule étape à l'aide de glutaraldéhyde décrit dans Immunochemistry, 1969, 6, 43—53.

Avant l'étape de couplage, les anticorps ont été dialysés pendant une nuit à 4°C contre un tampon phosphate 0,1 M ph 6,8. 30 µl d'une solution de glutaraldéhyde à 1% ont été ajoutés sous agitation douce à 1 ml d'un tampon phosphate 0,1 M pH 6,8 contenant 2 mg d'anticorps anti-globules rouges de mouton et 2 mg d'anticorps anti-immunoglobulines humaines. Après une incubation de 3 heures à la température ambiante, on a ajouté 50 µl d'une solution de glycine 2 M et, deux heures après, le mélange a été dialysé pendant une nuit à 4°C contre une solution physiologique tamponnée (tampon phosphate). Après centrifugation (3000 g, 30 minutes) et addition d'un égal volume de glycérol, la préparation obtenue a été stockée à −20°C jusquà son utilisation.

Des plaques de polystyrène ayant des puits en U ou en V ont été revêtues de DNA de thymus de veau par incubation de 0,2 ml d'une solution de DNA (0,5 mg/ml) par puits pendant 2 heures à 37°C et 16 heures à 4°C. Le DNA était en solution dans un tampon citrate 0,1 M pH 6,0 contenant 0,1 M de NaCl. 0,2 ml de solution du sérum à tester (contenant des anticorps anti-DNA] DANS DU PBS-TWEEN contenant 1% de sérumalbumine bovine a été ajoutée dans chaque puits revêtu de DNA et on a laissé incuber pendant 16 heures à 4°C. Les plaques ont été lavées 3 fois avec du PBS-TWEEN, et 0,2 ml du produit de couplage préparé comme ci-dessus a été ajouté dans les puits. Après incubation pendant 2 heures à 37°C, les plaques ont été lavées trois fois avec du PBS-TWEEN, et ensuite on a ajouté 0,2 ml d'une suspension à 0,1% de globules rouges de mouton et on a déterminé l'érythroadsorption. Avant leur utilisation, les globules rouges conservés dans le milieu stérile de Elsever ont été lavés 10 fois par centrifugation avec du PBS.

Cet essai a été réalisé avec différentes dilutions du sérum à tester et on a noté dernière dilution donant une érythroadsorption encore visible. Les résultats obtenus sont consignés dans le Tableau I ci-après.

### Exemple comparatif 1 bis

Un a réalisé le même dosage en utilisant comme produit de couplage le produit de couplage d'anticorps anti-immunoglobulines humaines et d'avidine obtenu selon le mode opératoire décrit ci-dessus en utilisant 30 µl d'une solution de glutaraldéhyde à 1%, et une solution de 1 ml de tampon phosphate 0,1 M pH 6,8 contenant 8 mg d'avidine et 2 mg d'anticorps anti-Ig humaines.

Dans cet essai, les érythrocytes ont été modifiés à l'aide de biotine selon le mode opératoire ci-après, analogue à celui décrit par JASIEWICZ et al. dans Expl. Cell. Res. 1976, 100, 213—217, en utilisant du biotinyl-N-hydroxysuccinimide. Des érythrocytes lavés au PBS (10 fois) ont été lavés 3 fois de plus avec une solution de 0,10 M NaCl contenant 0,05 M de NaHCO$_3$. Les érythrocytes ont été biotinylés par addition de 7 mg de biotinyl-N-hydroxysuccinimide dissous dans 80 μl de diméthylformamide distillé dans 2 ml d'une suspension à 2,5% d'érythrocytes. Ce mélange a été incubé pendant une heure à la température ambiante et lavé trois fois avec du PBS. Finalement, la suspension a été ajustée à 0,1% pour être utilisée dans le procédé. Les résultats obtenus sont consignés dans le tableau I ci-après.

### Exemble comparatif 1 ter

A titre de comparaison, on a réalisé le même dosage en utilisant comme produit de couplage le produit de couplage concanavaline A-anticorps anti-Ig humaines obtenu selon le procédé décrit dans la demande de brevet FR-8 011 470. Dans ce cas particulier, la solution du produit de couplage anticorps anti-Ig humaines utilisée dans l'étape 3 du procédé contenait du méthyl-alpha-D-mannoside pour éviter que la concanavaline réagisse avec des fractions glycosidiques présentes dans le sérum à tester et qui fausseraient l'érythroadsorption. Les résultats obtenus selon ce mode opératoire sont également consignés dans le tableau I.

En utilisant un mode opératoire analogue à celui décrit ci-dessus on a pu également doser des IgE humaines avec une très grande sensibilité.

### Exemple 2

### Comparaison avec le dosage immunoenzymatique

On a effectué le même dosage (anticorps anti-DNA) qu'à l'exemple 1, mais on a remplacé l'étape d'incubation avec le produit de couplage ligand spécifique-ligand capable de réagir avec des érythrocytes par une étape d'incubation avec un produit de couplage ligand spécifique-enzyme; après incubation puis lavage, on a déterminé l'activité enzymatique.

Comme enzymes, on a utilisé la glucose-oxydase (Aspergillus niger glucose oxydase (qualité 1) fournie par Boehringer—Mannheim, RFA). Le mode opératoire pour révéler cette enzyme est décrit en détail dans les articles ci-après:

— J. Histochem. Cytochem. 1979, 27, 1131 — 1139,
— J. Allergy Clin. Immunol. 1978, 61, 23 — 27.

Les résultats de ces dosages immunoenzymatiques sont consignés dans le tableau I.

Ces résultats montrent que le procédé de l'invention procure des résultats analogues à ceux obtenus avec le procédé de dosage immunoenzymatique. La spécificité du dosage a été démontrée par l'absence d'érythroadsorption lorsque le sérum contenant de très faibles quantités d'anticorps anti-DNA a été dosé, ou lorsqu'un sérum contenant des anticorps anti-DNA a été préalablement incubé avec un excès de DNA.

Tableau I
Dosage des anticorps anti-DNA humains par dosage immunoenzymatique selon le procédé de l'invention

| Dilution du sérum | Sérum humain normal dosage immuno-enzymati-que*) | **) Av | Ab | Con A | Sérum de malade dosage immuno-enzymati-que*) | **) Av | Ab | Con A |
|---|---|---|---|---|---|---|---|---|
| 1/50 | 0,66 | – | + | + | 3,00 | + + + | + + + + | + + + + |
| 1/150 | 0,56 | – | ± | + | 2,65 | + + | + + + | + + + |
| 1/450 | 0,33 | – | – | ± | 1,82 | – | + + | + + |
| 1/1350 | 0,10 | – | – | ± | 0,99 | – | ± | + |
| 1/4050 | 0,00 | – | – | – | 0,51 | – | – | ± |
| 1/12 150 | 0,00 | – | – | – | 0,14 | – | + | ± |
| 1/36 450 | 0,00 | – | – | – | 0,03 | – | – | – |
| témoin sans sérum | 0,00 | – | – | – | 0,00 | – | – | – |

*) réactif utilisé:
anticorps anti-immunoglobulines humaines marquées par la glucose oxydase.

**) réactif utilisé:
produit de couplage d'anticorps anti Ig humaines avec l'Avidine (Av), des anticorps anti-globules rouges de mouton (Ab) selon l'invention et de la Concanavaline A (Con A).

**Revendications**

1. Procédé de détection et de dosage d'une substance biologique par érythroadsorption, caractérisé en ce qu'il consiste:

(1) à immobiliser une substance ayant une affinité de fixation pour la substance biologique à doser;
(2) à faire incuber cette substance avec le milieu liquide contenant la substance biologique à doser;
(3) à faire incuber, après lavage, le milieu réactionnel résultant avec le produit de couplage d'un ligand spécifique de la substance à doser et d'un anticorps anti-érythrocytes,
(4) à ajouter des érythrocytes, et
(5) à déterminer l'érythroadsorption.

2. Procédé selon la revendication 1, caractérisé en ce que la substance biologique à doser est un antigène, un anticorps, un haptène, une hormone ou une immunoglobuline.

3. Procédé de détection selon l'une des revendications 1 ou 2, caractérisé en ce qu'on détermine l'érythroadsorption visuellement.

4. Procédé de dosage selon l'une quelconque des revendications 1 ou 2, caractérisé en ce qu'on détermine l'importance de l'érythroadsorption en réalisant une lyse des érythrocytes fixés sur le produit de couplage mis en oeuvre et en dosant une substance libérée, par les érythrocytes lysés.

5. Procédé selon l'une quelconque des revendications 1 à 4, caractérisé en ce que le milieu liquide contenant la substance biologique à doser est un sérum.

6. Procédé selon la revendication 5, caractérisé en ce que le sérum est au préalable absorbé par des globules rouges.

7. Procédé selon la revendication 6, caractérisé en ce que l'absorption du sérum par des globules rouges est réalisée par les étapes suivantes qui consistent:

a) à décomplémenter le sérum, par chauffage à 56° C pendant environ 30 minutes, si la substance à doser est stable,
b) à ajouter un excès de globules rouges de mouton audit sérum, par exemple pour obtenir une concentration de 10% de globules rouges;

c)  à laisser le mélange de sérum et globules à la température ambiante pendant environ 1 heure sous agitation douce;

d)  à centrifuger pour récupér le sérum purifié et éliminer le culot de globules rouges.

8. Procédé selon la revendication 7, caractérisé en ce que le sérum est absorbé par des globules rouges traités par un agent tannant, tel que le glutaraldéhyde.

9. A titre de réactif de dosage pour le procédé selon la revendication 1, le produit de couplage d'un ligand spécifique de la substance à doser et d'un anticorps anti-érythrocytes.

10. Réactif selon la revendication 9, caractérisé en ce que le ligand spécifique est un anticorps ou un antigène.

## Patentansprüche

1. Verfahren zum Nachweisen und Bestimmen einer biologischen Substanz durch Erythroadsorption, dadurch gekennzeichnet, daß es besteht aus:

(1)  Immobilisieren einer Substanz, die eine Fixierungsaffinität für die zu bestimmende biologische Substanz hat;

(2)  Inkubieren dieser Substanz mit dem flüssigen Medium, das die zu bestimmende biologische Substanz enthält;

(3)  Inkubieren, nach dem Waschen, des erhaltenen Reaktionsmediums mit dem Kupplungsprodukt eines für die zu bestimmende Substanz spezifischen Liganden und eines Anti-Erythrocyten-Antikörpers;

(4)  Zugeben von Erythrocyten und

(5)  Bestimmen der Erythroadsorption.

2. Verfahren nach Anspruch 1, dadurch gekennzeichnet, daß die zu bestimmende biologische Substanz ein Antigen, ein Antikörper, ein Hapten, ein Hormon oder ein Immunoglobulin ist.

3. Nachweisverfahren nach einem der Ansprüche 1 oder 2, dadurch gekennzeichnet, daß man die Erythroadsorption visuell bestimmt.

4. Bestimmungsverfahren nach irgendeinem der Ansprüche 1 oder 2, dadurch gekennzeichnet, daß man das Ausmaß der Erythroadsorption dadurch bestimmt, indem man eine Lyse der auf dem eingesetzten Kupplungsprodukt fixierten Erythrocyten durchführt und eine von den lysierten Erythrocyten freigesetzte Substanz bestimmt.

5. Verfahren nach irgendeinem der Ansprüche 1 bis 4, dadurch gekennzeichnet, daß das flüssige Medium, das die zu bestimmende biologische Substanz enthält, ein Serum ist.

6. Verfahren nach Anspruch 5, dadurch gekennzeichnet, daß das Serum vorher durch rote Blutkörperchen absorbiert wird.

7. Verfahren nach Anspruch 6, dadurch gekennzeichnet, daß die Absorption des Serums durch rote Blutkörperchen in den folgenden Schritten durchgeführt wird:

(a)  Dekomplementieren des Serums durch Erhitzen auf 56° C während ungefähr 30 Minuten, wenn die zu bestimmende Substanz stabil ist;

(b)  Zugabe eines Überschusses von roten Hammel-Blutkörperchen zu dem Serum, um z. B. eine Konzentration der roten Blutkörperchen von 10% zu erhalten;

(c)  Belassen des Gemisches aus Serum und Blutkörperchen bei Umgebungstemperatur während ungefähr 1 Stunde unter leichtem Rühren;

(d)  Zentrifugieren, um das gereinigte Serum zu gewinnen und den Bodensatz von roten Blutkörperchen abzutrennen.

8. Verfahren nach Anspruch 7, dadurch gekennzeichnet, daß das Serum durch rote Blutkörperchen absorbiert wird, die mit einem Gerbmittel, wie Glutaraldehyd, behandelt worden sind.

9. Als Bestimmungsreagenz für das Verfahren nach Anspruch 1 das Kupplungsprodukt eines für die zu bestimmende Substanz spezifischen Liganden und eines Anti-Erythrocyten-Antikörpers.

10. Reagenz nach Anspruch 9, dadurch gekennzeichnet, daß der spezifische Ligand ein Antikörper oder ein Antigen ist.

## Claims

1. Method of detecting and determining a biological substance by erythroadsorption, characterized in that it consists in:

(1)  immobilizing a substance having a fixing affinity for the biological substance to be determined;

0 055 751

(2) incubating this substance with the liquid medium containing the biological substance to be determined;

(3) incubating the resulting reaction medium, after washing, with the coupled product of a specific ligand for the substance to be determined and an antibody to erythrocyte

(4) adding erythrocytes and

(5) determining the erythroadsorption.

2. Method according to Claim 1, characterized in that the biological substance to be determined is an antigen, an antibody, a haptene, a hormone or an immunoglobulin.

3. Method of detection according to either claim 1 or 2, characterized in that the erythroadsorption is determined visually.

4. Method of determination according to either Claim 1 or 2, characterized in that the magnitude of the erythroadsorption is determined by carrying out a lysis of the erythrocytes fixed to the coupling product and determining a substance released by the lysed erythrocytes.

5. Method according to any of claims 1 to 4, characterised in that the liquid medium containing the biological substance to be determined is a serum.

6. Method according to claim 5, characterized in that the serum is beforehand absorbed by red blood cells.

7. Method according to claim 6, characterized in that the absorption of the serum by the red blood cells consists of the following steps,

a) decomplementing the serum by heating at 56°C for about 30 minutes, if the substance to be determined is stable,

b) adding an excess of sheep red blood cells to the said serum, for example to achieve a red blood cells concentration of 10%

c) leaving the mixture of serum and red cells at ambient temperature for about 1 hour, with gentle stirring and

d) centrifuging, to recover the purified serum and remove the bottom of red blood cells.

8. Method according to claim 7, characterized in that the serum is absorbed by red blood cells treated with a tanning agent, such as glutaraldehyde.

9. By way of a reagent for the method according to claim 1, the coupled product of a specific Ligand for the substance to be determined and an antibody to erythrocyte.

10. Reagent according to claim 9, characterized in that the specific Ligand is an antibody or an antigen.

9

FIG.1